# EUROPEAN PATENT SPECIFICATION

(11) **EP 0 425 963 B1**
(45) Date of publication and mention of the grant of the patent: **15.06.1994**
(21) Application number: 90120227.5
(22) Date of filing: 22.10.1990
(51) Int. Cl.: A61M 25/00

(54) **Device for conveying blood flow during liver surgery**
Vorrichtung zur Blutstromförderung während einer Leberoperation
Dispositif pour assurer la circulation sanguine pendant la chirurgie hépatique

(30) Priority: 31.10.1989 IT 2220489
(43) Date of publication of application: 08.05.1991
(73) Proprietor: DIDECO S.p.A., I-41037 Mirandola (province of Modena) (IT)
(72) Inventor: Bigi, Leonardo, I-41037 Mirandola (Modena) (IT)
(74) Representative: Modiano, Guido, Dr.-Ing.

(56) References cited:
- EP-A- 0 159 773
- EP-A- 0 212 159
- US-A- 3 851 646
- US-A- 4 592 754

## Description

The invention relates to a device for conveying blood flow during liver surgery.

It is known that surgical operations involving the liver of a patient, both those which provide its removal and those during which it is necessary to temporarily bypass the hepatic vascular system, have numerous problems.

The technique which entails the clamping of the vena portae and the superior vena cava during surgery causes the unfavorable condition of marked renal and intestinal hypertension with possible severe clinical consequences.

In order to obviate this situation, attempts have been made to establish an extra-corporeal circulation, but with such methods as to cause hardly negligible technical and logistic problems.

Another unfavorable circumstance which arises in the known technique resides in the fact that the extensive bleeding produced forces very fast surgical actions on the part of the surgeon and the adoption of auto-transfusion, with all the problems which arise therefrom.

EP-A-0 212 159 shows a catheter device for blood flow which comprises an elongated tube perforated at one end and connected at another end to a blood circuit. A plunger element is also provided which is slidingly movable inside the tube in order to either block or open the perforations in the end of the tube.

The aim of the present invention is to provide a device for conveying blood flow during liver surgery which allows to eliminate all clinical problems for the patient, which allows the surgeon to operate in the most favorable conditions and has maximum simplicity.

The proposed aim is achieved by a device for conveying blood flow during liver surgery, according to the invention, as defined in the appended claim 1.

Further characteristics and advantages will become apparent from the description of a preferred but not exclusive embodiment of the invention, illustrated only by way of non-limitative example in the accompanying drawings, wherein:
figure 1 is a partially sectional view of the invention;
figure 2 is a view of the invention during an application.

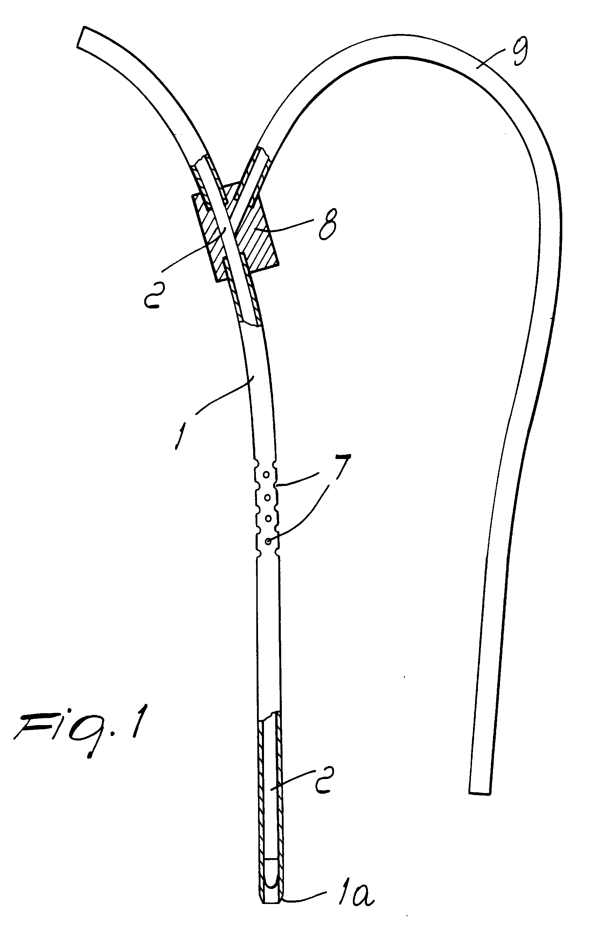

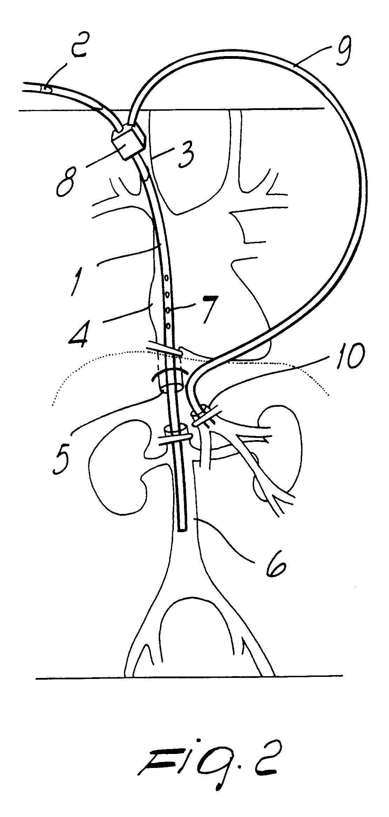

With particular reference to the above figures, the reference numeral 1 indicates the first tubular element, made of any polymeric material with a good degree of biocompatibility, such as for example polyurethane, internally provided with the sealingly slidable obturator 2, which is illustrated in figure 1 in the position it assumes during the insertion of the element 1 in the patient's body, whereas in figure 2 it is illustrated in the position it assumes during surgery, as will be better explained hereinafter.

Said tubular element 1 is adapted to be inserted in the patient's body passing through the right external jugular vein 3, the right cardiac atrium 4 and the superior vena cava 5 to be connected to the inferior vena cava 6 during surgery requiring the removal of the liver, which is indeed interposed between said veins 5 and 6, or the temporary bypassing of the liver vascular system.

The element 1 is provided, in the wall portion intended to be comprised inside the cardiac atrium 4, with through holes such as 7 which return blood to the heart.

The connection 8 to the second tubular element 9 is provided on said element 1 in such a position as to be external to the body of the patient when the first tubular element 1 is completely inserted; said tubular element 9 is adapted to be connected to the vena portae 10 after defining a path outside the patient's body.

The constructive details to be observed are constituted by the tapered shape, such as 1a, of the ends of the tubular elements, and by the fact that the continuity of the internal surfaces of the walls of said tubular elements is maintained inside the connection 8 so as to avoid any trauma to the blood which affects them.

The operation of the invention is evident.

The tubular element 1 is inserted in the right jugular vein 3 through an incision in the patient's neck, with the obturator 2 protruding from the ends 1a, and is then inserted in the right cardiac atrium 4 and in the superior vena cava 5 to be connected to the lower vena cava 6.

The connection 8 has remained external to the patient's body, and the second tubular element 9, after a certain external path, is also inserted in the patient's body and is connected to the vena portae 10.

When the entire apparatus is connected by ligature of the tubular elements to the veins, the obturator 2 is retracted until it reaches the position of figure 2, and in this manner the return of venous blood from the lower caval system and from the portal system is re-established even when the liver is removed or the vascular system thereof is bypassed.

From what has been described the advantageous characteristics of the invention are evident; the invention allows, with a very simple device, to maintain high blood flows from the vena portae and from the inferior vena cava to the right cardiac atrium while the liver is bypassed from circulation, so as to clinically protect the patient, and furthermore allows the surgeon to operate with maximum comfort.

In the practical embodiment of the invention, the materials employed, as well as the shapes and dimensions, may be any according to the requirements.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Device for conveying blood flow during liver surgery, comprising a first tubular element (1) having a first end (1a), a second end, and a middle wall portion between said two ends, through holes (7) for the passage of blood being provided in said first tubular element, the device further comprising an obturator (2) which is sealingly slidable inside said tubular element from said second end between a closed position which blocks said through holes (7) and an open position in which said through holes are open, the device being characterized in that a first end (1a) of said two ends is open and said through holes are provided in said middle wall portion away from said first end, the device further comprising a second tubular element (9) having a first end which is connected by a connector (8) to said first tubular element at a position between said second end of said first tubular element and said through holes, said second tubular element having a second end which is open.

2. Device according to claim 1, characterized in that said ends (1a) of the two tubular elements (1,9) are tapered.

3. Device according to claims 1 or 2, characterized in that a continuity of the inner surfaces of the walls of the two tubular elements (1,9) is present inside the connection (8) between said tubular elements.

## Patentansprüche

1. Vorrichtung zur Blutstromförderung während einer Leberoperation, umfassend ein erstes Röhrenelement (1) mit einem ersten Ende (1a), einem zweiten Ende und einem mittleren Wandteil zwischen den beiden Enden, und Durchgangslöchern (7) für den Durchgang von Blut, welche in dem ersten Röhrenelement vorgesehen sind, wobei die Vorrichtung weiterhin eine Schließvorrichtung (2) umfaßt, welche dichtend in dem Röhrenelement von dem zweiten Ende zwischen einer geschlossenen Stellung, welche die Durchgangslöcher (7) blockiert, und einer offenen Stellung, in welcher die Durchgangslöcher offen sind, gleitbar ist, wobei die Vorrichtung **dadurch gekennzeichnet ist, daß** ein erstes Ende (1a) der beiden Enden offen ist und die Durchgangslöcher in dem mittleren Wandteil im Abstand von dem ersten Ende vorgesehen sind, wobei die Vorrichtung weiterhin ein zweites Röhrenelement (9) mit einem ersten Ende umfaßt, welches durch einen Verbinder (8) mit dem ersten Röhrenelement an einer Stelle zwischen dem zweiten Ende des ersten Röhrenelementes und den Durchgangslöchern verbunden ist, wobei das zweite Röhrenelement ein zweites Ende aufweist, welches offen ist.

2. Vorrichtung nach Anspruch 1, **dadurch gekennzeichnet, daß** die Enden (1a) des zweiten Röhrenelementes (1,9) verjüngt sind.

3. Vorrichtung nach Anspruch 1 oder 2, **dadurch gekennzeichnet, daß** eine Kontinuität der inneren Flächen der Wände der zwei Röhrenelemente (1,9) innerhalb des Verbinders (8) zwischen den beiden Röhrenelementen vorhanden ist.

## Revendications

1. Dispositif pour assurer la circulation sanguine pendant la chirurgie du foie, comprenant un premier élément tubulaire (1) ayant une première extrémité (1a), une seconde extrémité, et une partie de paroi médiane entre les deuxdites extrémités, des trous traversants (7) pour le passage du sang étant prévus dans ledit premier élément tubulaire, le dispositif comprenant, de plus, un obturateur (2) qui est coulissant, de façon étanche, à l'intérieur dudit élément tubulaire à partir de ladite seconde extrémité, entre une position fermée qui bloque lesdits trous traversants (7) et une position ouverte dans laquelle lesdits trous traversants sont ouverts, le dispositif étant caractérisé en ce que la première extrémité (1a) des deuxdites extrémités est ouverte et lesdits trous traversants sont prévus dans ladite partie de paroi médiane, éloignés de ladite première extrémité, le dispositif comprenant de plus un second élément tubulaire (9) ayant une première extrémité qui est reliée par une liaison (8) audit premier élément tubulaire, à une position entre ladite seconde extrémité dudit premier élément tubulaire et lesdits trous traversants, ledit second élément tubulaire ayant une seconde extrémité qui est ouverte.

2. Dispositif selon la revendication 1,
caractérisé en ce que lesdites extrémités (1a) des deux éléments tubulaires (1,9) sont coniques.

3. Dispositif selon les revendications 1 ou 2,
caractérisé en ce qu'une continuité des surfaces internes des parois des deux éléments tubulaires (1,9) est présente à l'intérieur de la liaison (8) entre lesdits éléments tubulaires.
